Europäisches Patentamt

European Patent Office

Office européen des brevets

(19)

(11) Veröffentlichungsnummer: **0 103 753**
**B1**

(12) **EUROPÄISCHE PATENTSCHRIFT**

(45) Veröffentlichungstag der Patentschrift:
08.06.88

(21) Anmeldenummer: 83108151.8

(22) Anmeldetag: 18.08.83

(51) Int. Cl.⁴: **B 65 F 1/16,** B 65 F 1/14,
B 65 F 1/06, B 65 D 43/12

(54) Hygienebehälter.

(30) Priorität: 20.08.82 DE 3230954
02.02.83 DE 8302763 U
15.02.83 DE 8304106 U

(43) Veröffentlichungstag der Anmeldung:
28.03.84 Patentblatt 84/13

(45) Bekanntmachung des Hinweises auf die Patenterteilung:
08.06.88 Patentblatt 88/23

(84) Benannte Vertragsstaaten:
AT BE CH DE FR GB IT LI LU NL SE

(56) Entgegenhaltungen:
EP-A-0 003 580
BE-A-860 590
DE-A-2 024 421
DE-A-3 241 146
FR-A-477 839
FR-A-1 538 625
GB-A-200 569
GB-A-432 862
GB-A-566 186
GB-A-948 898
US-A-3 847 332

(73) Patentinhaber: Calmic GmbH, Gutenbergstrasse 4,
D-6457 Maintal- Bischofsheim (DE)

(72) Erfinder: Andrews, Stanley D., Uhlandstrasse 10,
D-6454 Bruchköbel (DE)

(74) Vertreter: Stoffregen, Hans- Herbert, Dr. Dipl.-
Phys., Patentanwälte Strasse & Stoffregen
Salzstrasse 11a Postfach 2144, D-6450
Hanau/Main 1 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die Erfindung bezieht sich auf einen Hygienebehälter, beispielsweise zur Aufnahme von Binden, Tampons oder ähnlichem, der mit einem vorzugsweise eine verschwenkbare, eine Einfüllrutsche aufweisende Klappe umfassenden Deckel verschließbar ist, wobei der Behälter an zwei gegenüberliegenden Seiten Einbuchtungen aufweist, in denen Abschnitte des Deckels einbringbar sind.

Ein entsprechender Hygienebehälter ist zum Beispiel dem DE-U-7 803 139 (EP-A-0 003 580) zu entnehmen. Er besteht im wesentlichen aus einem gegen chemische Einflüsse resistenten Kunststoffgehäuse, das mit dem vorzugsweise aus korrosionsbeständigen metallischen Werkstoffen bestehenden Deckel verschließbar ist. Die in der Einfüllklappe vorhandene Einfüllrutsche hat dabei die Funktion, daß selbst bei angehobener Klappe das Behälterinnere nicht ohne weiteres zugänglich ist. Um Binden oder ähnliches in den Behälter einzubringen, müssen diese zunächst in die Einfüllrutsche gelegt werden, von der sie bei auf dem Deckel aufliegender Klappe in das Behälterinnere fallen. Der Deckel selbst ist mit zwei umgebogenen Blechzungen versehen, welche in am oberen Rand des Behälters vorhandene Ausnehmungen einrasten. Damit der Behälter entleert und gereinigt werden kann, muß der Deckel entfernt werden. Dies erfolgt entweder dadurch, daß die die Ausnehmungen aufweisenden Längsseiten des Behälters eingedrückt werden, was nur unter erhöhtem Kraftaufwand möglich ist. Gleichzeitig erfolgt dadurch eine Deformation des Behälters, die zu einer Beschädigung führen kann. Alternativ dazu können mit einem Schraubenzieher oder einem ähnlichen Gegenstand die umgebogenen Blechzungen über den oberen Behälterrand gehoben werden. Diese Handhabung kann zu einer Beschädigung des Behälters und/oder Deckels führen. Ferner kann zum Beispiel beim Abrutschen des Werkzeuges das Bedienungspersonal verletzt werden. Ein weiterer Nachteil in der Ausbildung bekannter Hygienebehälter ist darin zu sehen, daß die Ränder des Deckels über die Seitenflächen des Behälters ragen, wodurch ein erhöhter Platzbedarf beim Transport und beim Aufstellen notwendig ist. Letzteres führt auch häufig dazu, daß insbesondere in schmalen Damentoiletten entsprechende Behälter nicht eingesetzt werden können. Ferner besteht normalerweise keine Möglichkeit oder nur mit einer konstruktiv aufwendigen Halterung, die Behälter an einer Wand zu befestigen, da erwähntermaßen die Deckelränder über die Behälteraußenwandung vorstehen. Um einen die Binden u.ä. aufnehmenden Beutel in dem Behälter anzuordnen, wird der obere Beutelrand um den Behälterrand umgeschlagen. Durch das Aufbringen des Deckels wird dann der Beutel festgelegt, wobei jedoch Abschnitte von diesem sichtbar bleiben. Dieses wirkt optisch unschön.

Ferner kann beim Entfernen des Deckels der Beutelrand nach innen gleiten, wodurch dieser unerwünschterweise beschmutzt wird.

Aufgabe der vorliegenden Erfindung ist es, einen Hygienebehälter der eingangs genannten Art so auszubilden, daß ein problemloses Verschließen bei gleichzeitiger Gewähr, daß ein unkontrolliertes Entfernen des Deckels nicht möglich ist, gegeben ist, daß der Behälter platzsparend ausgebildet und damit auch auf engstem Raum angeordnet und daß der Behälter optisch einen ansprechenden Eindruck vermittelt.

Die Aufgabe wird dadurch gelöst, daß die Einbuchtungen Führungen sind, in denen die Abschnitte verschiebbar sind, daß die Ränder oder zumindest Abschnitte der Ränder des Deckels derart abgewinkelt sind, daß die Ränder bei auf dem Behälter aufgebrachtem Deckel fluchtend oder nahezu fluchtend zu den entsprechenden Seitenflächen des Behälters ausgerichtet sind, und daß von der Deckelinnenfläche ein Arretierelement ausgeht, das in Form einer blattfederartigen Zunge mit gegebenenfalls abgewinkeltem freien Ende im vorderen Bereich des Deckels angeordnet ist, in den Behälter hineinragt und bei den Behälter verschließendem Deckel im Bereich des Randes der vorderen Seitenfläche des Behälters derart zu liegen kommt, daß ein Herausziehen des Deckels unterbunden ist.

Durch den erfindungsgemäßen Vorschlag wird demzufolge ein Hygienebehälter zur Verfügung gestellt, der ein einfaches Aufbringen des Deckels ermöglicht, indem dieser mit Randabschnitten in Führungen eingebracht und entlang dieser verschoben wird. Dabei sind die Führungen parallel zu dem oberen Rand des Behälters verlaufende Ausnehmungen in Form von Nuten, in die einander zugewandte Abschnitte der abgewinkelten Seitenränder des Deckels einbringbar sind. Durch das Arretierelement, das eine blattförmige Zunge ist, dessen vorderes Ende bei den Behälter verschließendem Deckel mit dem Rand der Vorderfläche des Behälters in Wechselwirkung tritt, ist sichergestellt, daß ein unkontrolliertes oder ungewünschtes Verschieben nicht möglich ist. Dabei ist das blattfederartige Element zwischen Klappe und vorderem Rand des Deckels angeordnet. Das Bedienungspersonal kann den Deckel dadurch entfernen, daß zum Beispiel eine Hand bei halbgeöffneter Klappe das Arretierelement in Richtung der Deckelinnenfläche drückt, so daß die Arretierung aufgehoben ist.

Um beim Betätigen der Klappe auszuschließen, daß der Deckel wackelt, weisen die Führungen zumindest jeweils einen ihre lichte Weite verringernden Vorsprung auf, wobei der Abstand zwischen Vorsprung und angrenzender Führungswandung gleich oder geringfügig geringer als die Stärke des abgewinkelten Randabschnittes des Deckels ist.

Nach einer weiteren Ausführungsform der Erfindung weist die dem Behälterinneren zugewandte Hauptfläche des Deckels einen Abstand zum oberen Rand des Behälters auf, um dadurch bei an die Innenfläche gedrücktem Arretierelement ein einfaches Entfernen zu ermöglichen.

Ist die sichtbare Erstreckung des vorderen und der seitlichen abgewinkelten Randabschnitte des Deckels gleich oder in etwa gleich groß, so ist der hintere Randabschnitt gegenüber diesen um den Abstand verringert, der in etwa gleich dem Abstand zwischen oberem Rand des Behälters und der an diesen angrenzenden Wandung der Führung bzw. der Nut. Dadurch ergibt sich auch im hinteren Bereich ein glatter oder nahezu glatter Abschluß zwischen Behälterwandung und Deckel.

Um auch im vorderen Bereich einen bündigen Übergang zwischen Deckel und Behälterwandung zu erzielen, weist die entsprechende Behälterwandung einen Absatz auf, dessen Tiefe gleich oder in etwa gleich der Stärke des abgewinkelten Deckelmaterials ist. Die Höhe der Stufe entspricht der Abmessung des die Behälterwandung umgreifenden Deckelrandabschnitts.

Durch den bündigen Übergang zwischen Deckel und Behälteraußenwandung ergibt sich der Vorteil, daß ein geringerer Raumbedarf gegenüber den bekannten Behältern erforderlich und daß außerdem die Möglichkeit einer Befestigung mittels einer Wandhalterung gegeben ist, so daß der Behälter vom Boden beabstandet ist. Durch den fluchtend oder nahezu fluchtend erfolgenden Übergang zwischen Behälteraußenwandung und der diese teilweise umfassenden Randabschnitte des Deckels ergibt sich außerdem ein überaus gefälliges Äußeres. Aufgrund des Arretierelementes ist außerdem sichergestellt, daß bei der Benutzung des Behälters ein ungewolltes bzw. unkontrolliertes Entfernen des Deckels nicht möglich ist.

Um den die Binden o.ä. aufnehmenden Beutel einfach, jedoch sicher in dem Behälter anordnen zu können, ohne daß Randabschnitte sichtbar bleiben oder beim Entfernen des Deckels die Gefahr besteht, daß der Beutel unkontrolliert nach innen rutscht, wird vorgeschlagen, daß der Beutel von in zumindest zwei gegenüberliegenden Seite des Behälters lösbar angeordneten Halteelementen festgelegt ist, die in zumindest zwei gegenüberliegenden parallel oder nahezu parallel zur Behälteröffnung verlaufenden Längsschlitzen eingebracht sind, die ihrerseits durch die nach innen abgewinkelten parallel oder nahezu parallel zur Deckelfläche verlaufenden Behälterränder und die parallel dazu in den zwei gegenüberliegenden Seiten verlaufenden, durch die Führungen für die Behälterdeckel-Randabschnitte vorgegebenen Einbuchtungen gebildet sind. Demzufolge wird erfindungsgemäß vorgeschlagen, daß der mit dem Abfall in Berührung kommende Innenraum durch den entfernbaren Beutel oder Sack derart ausgeschlagen wird, daß ein sicheres Festlegen im oberen Behälterrand gewährleistet ist, ohne daß Beutelabschnitte von außen her sichtbar bleiben und beim Entfernen des Deckels die Gefahr besteht, daß der Beutel nach innen gleitet. Zu diesem Zweck ist das Halteelement in zumindest zwei gegenüberliegenden parallel oder nahezu parallel zur Behälteröffnung verlaufenden Längsschlitzen eingebracht und ist als ein U-förmig ausgebildetes federndes Bügelelement ausgebildet. Um das Federelement leicht erfassen zu können, sollten die freien Schenkel des Bügelelementes erfaßbare Abschnitte aufweisen. Dies kann zum Beispiel in Form von umgebogenen Schenkelenden erfolgen, die also einen Abschnitt aufweisen, die in das Behälterinnere ragen und der zum Beispiel jeweils durch einen Finger erfaßbar ist.

Weitere Einzelheiten, Vorteile und Merkmale der Erfindung ergeben sich aus dem in der Zeichnung dargestellten bevorzugten Ausführungsbeispiel.

Es zeigen:

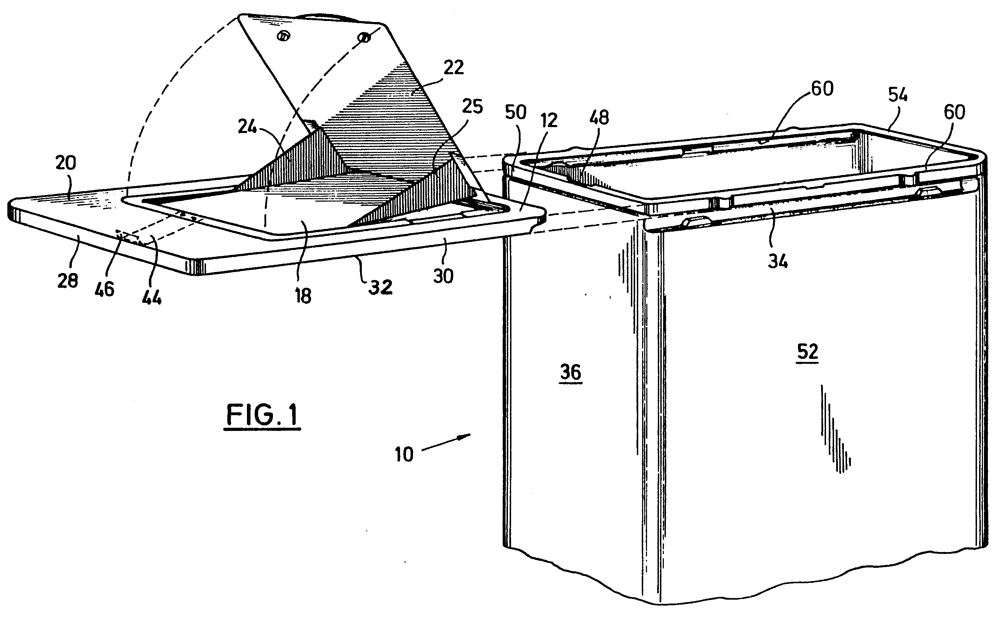

Fig. 1 einen erfindungsgemäßen Hygienebehälter in Explosionsdarstellung,
Fig. 2 eine Seitenansicht eines erfindungsgemäßen Behälters, teilweise im Schnitt,
Fig. 3 eine Vorderansicht des erfindungsgemäßen Behälters, gleichfalls teilweise im Schnitt,
Fig. 4 eine Schnittdarstellung des Hygienebehälters nach Fig. 1 mit eingebrachtem Beutel und
Fig. 5 ein für den in den Hygienebehälter eingebrachten Beutel bestimmtes Halteelement.

Der in den Figuren dargestellte Hygienebehälter 10, der vorzugsweise eine quaderförmige Form zeigt und aus gegenüber chemischen Einflüssen resistentem Kunststoff besteht, ist von einem Deckel 12 verschließbar, der seinerseits vorzugsweise aus metallischem, ebenfalls korrosionsbeständigem Werkstoff hergestellt ist. Der Deckel 12 zeigt in seinem Mittenbereich eine Vertiefung 14, in die eine schwenkbare Einfüllklappe 22 mit Einfüllrutsche 18 derart eingebracht ist, daß die Deckelhauptfläche 20 fluchtend in die Oberfläche der Klappe 22 übergeht. Ferner weist die Klappe 22 einen Griff 24 auf, um ein Verschwenken zu ermöglichen. Die Einfüllrutsche 18 besitzt taschenartige Seitenteile 24 und 25, die in ihren Endbereichen in die Ebene der Einfüllklappe 18 umgebogene, nicht näher dargestellte Laschen aufweisen und vorzugsweise mit korrespondierenden Abwinklungen der Einfüllklappe zu einer Einheit verbunden sind. Die Schwenklagerung der Klappe 22 erfolgt vorzugsweise dadurch, daß die hintere Kante der Klappe 22 eine Einrollung 27 zur Bildung einer Scharnierhälfte aufweist, die in eine von der Hauptfläche 20 ausgehende Lasche 31 als Gegenlager zu der Einrollung 27 vorhanden ist.

Der Deckel 12 weist ferner gegenüber der Hauptfläche um 90° oder nahezu 90° abgewinkelte Randabschnitte 28 und 30 und 42 auf, von denen die seitlichen Randabschnitte 30 in einander zugewandte parallel oder in etwa parallel zu der Deckeloberfläche 12 verlaufende Endbereiche 32 übergehen, die in Führungsschlitzen 34 einbringbar sind, die im Abstand zum oberen Rand des Behälters 10 und parallel dazu verlaufen. In diese vorzugsweise als Nuten ausgebildete Führungen 34 werden die abgewinkelten Enden 32 eingebracht, so daß der Deckel 12 ohne Schwierigkeiten von vorne auf den Behälter geschoben werden kann. Dabei geht die Führung 34 von der Vorderfläche 36 des Behälters 10 aus und endigt in einem Abstand zu der Rückfläche 38.

In der Führung 34 sind ferner Vorsprünge 40 vorgesehen, durch die der lichte Abstand der Führungswandungen abschnittweise verringert wird. Dadurch ist sichergestellt, daß der Deckel 12 ohne großes Spiel auf den Behälter 10 aufgesetzt ist, also insbesondere bei Betätigen der Klappe 22 nicht oder nur im geringen Umfang wackelt.

Um ein unkontrolliertes bzw. unbeabsichtigtes Entfernen des auf dem Behälter 10 aufgebrachten Deckels 12 zu verhindern, weist dieser zwischen der Klappe 22 und dem vorderen Rand 28 ein Arretierelement 44 in Form einer blattfederartigen von der Innenfläche des Deckels abragende Zunge 44 auf, die vorzugsweise mit einem abgewinkelten freien Ende 46 versehen ist, das bei aufgebrachtem Deckel 12 mit dem Rand 48 der Vorderfläche 36 des Behälters derart zusammenwirkt, daß ein Herausziehen des Deckels 12 blockiert ist. Um jedoch ein Entleeren des Behälters 10 zu ermöglichen, muß die Klappe 22 geringfügig angehoben werden, damit zum Beispiel eine Hand das Arretierelement 44 in Richtung der Innenfläche des Deckels 12 wegbiegt, so daß dadurch die Arretierung aufgehoben wird. Sodann kann der Deckel 12 ohne Schwierigkeiten aus der Führung 34 herausgezogen werden.

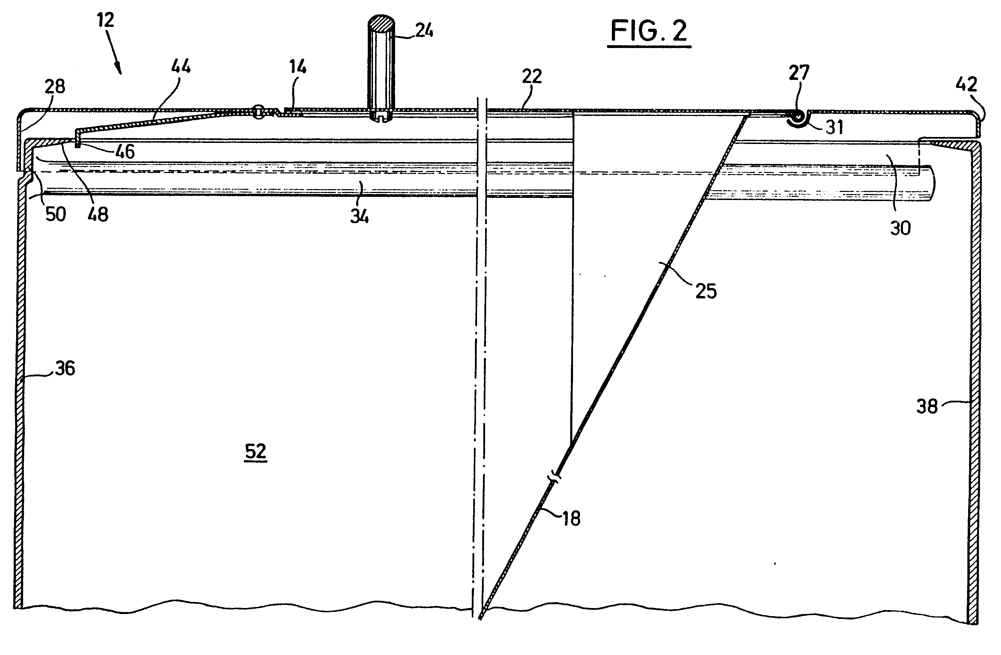

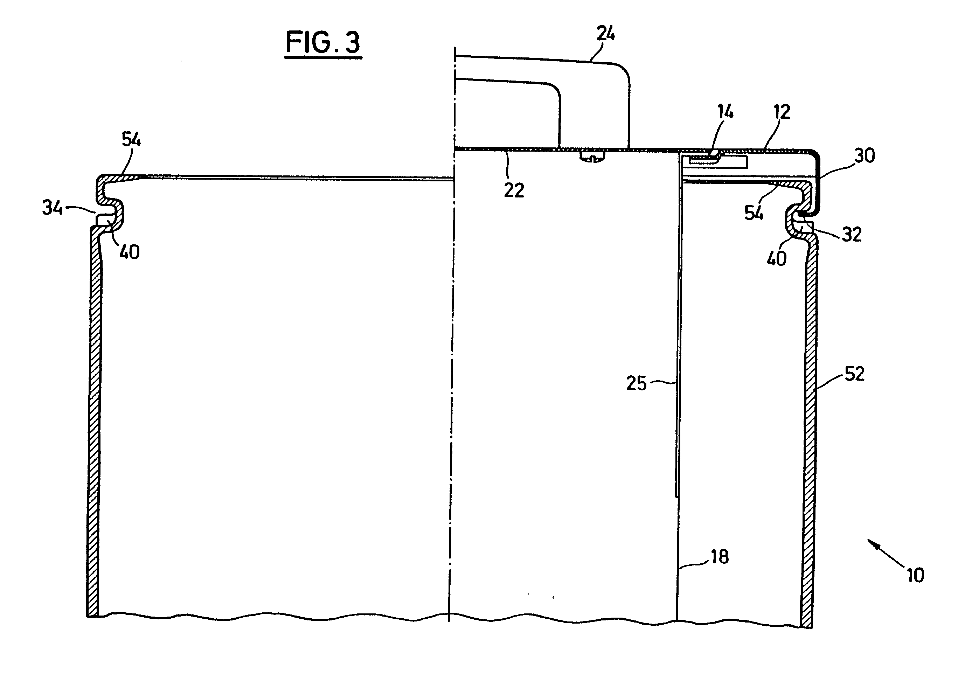

Wie insbesondere die Fig. 2 und 3 erkennen lassen, gehen die Randabschnitte 28, 30 und 42 bei auf dem Behälter 10 aufgebrachtem Deckel 12 bündig in die Behälteraußenwandungen über, haben also im Vergleich zu den bekannten Hygienebehältern keine vorstehenden Ränder. Um einen bündigen Übergang zu ermöglichen, weist die Vorderfläche 36 des Behälters 10 im Bereich des Deckelrandes 28 eine stufenförmig ausgebildete Ausnehmung 50 auf, deren Tiefe in etwa der Materialstärke des Randabschnitts 28 entspricht. Die Höhe der Stufe 50 kommt in etwa dem den Behälter 10 umfassenden Bereich des Randabschnitts 28 gleich. Ist an der Vorderfläche eine Stufe 50 zur Erzielung eines fluchtend oder nahezu fluchtenden Übergangs zwischen dem abgewinkelten Randabschnitt 28 und der Vorderfläche 36 ausgebildet, so ist hinsichtlich der Seitenflächen 52 der Bereich des Behälters 10 oberhalb der Führung 34 zurückversetzt, wie die Fig. 3 erkennen läßt. Dadurch ist gleichfalls sichergestellt, daß der parallel zur Wandung 52 verlaufende abgewinkelte Randabschnitt 30 in diesen bündig oder nahezu bündig übergeht.

Da der Deckel 12 im Abstand zu dem oberen Behälterrand 54 verläuft, der einen umlaufenden Bund darstellt, ist die Höhe des Randabschnitts 42 gleich oder geringer als der Abstand zwischen dem umlaufenden Rand 54 und der dem Behälterinneren zugewandten Fläche des Deckels 12. Dadurch ist gleichfalls gewährleistet, daß der Übergang zwischen der Rückwand 38 und dem abgewinkelten Rand 42 glatt oder nahezu glatt erfolgt.

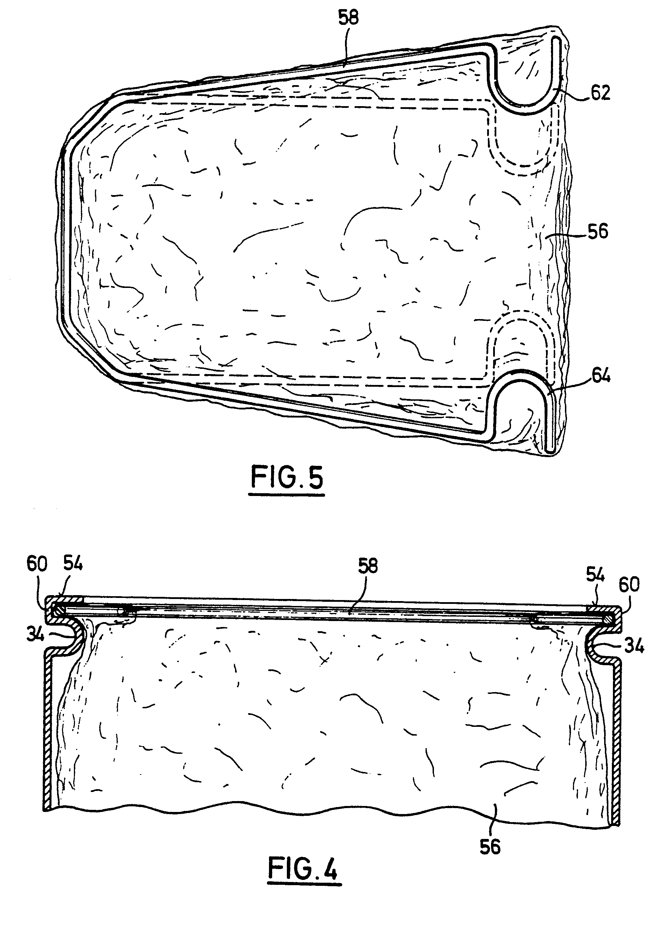

In dem Behälterinneren ist ein in den Fig. 4 und 5 beispielhaft dargestellter Beutel 56 oder Sack lösbar angeordnet, um den über die Rutsche eingebrachten Abfall aufzunehmen und somit ein einfaches Entleeren des Behälters 10 zu ermöglichen. Der Beutel 56 wird dabei von einem Halteelement 58 erfaßt, das selbst in im oberen Randbereich des Behälters vorhandene Längsschlitze 60 festlegbar ist. Diese Längsschlitze 60 werden im vorliegenden Fall durch den Zwischenraum zwischen den abgewinkelten oberen Rändern 54 des Behälters 10 sowie durch die von den Führungsschlitzen 34 in das Behälterinnere ausgeformten Ausbuchtungen gebildet. Dadurch ergibt sich der Vorteil, daß für das Halteelement 58 nicht zusätzliche im Behälterinneren vorzusehende Befestigungseinrichtungen angeordnet werden müssen. Ferner ist der Bügel im oberen Behälterbereich, also unmittelbar unterhalb des Deckels festgelegt, so daß sichergestellt ist, daß der gesamte Innenraum des Behälters 10 durch den Beutel bzw. Sack 56 abgedeckt ist.

Das Halteelement selbst stellt ein U-förmig ausgebildetes federndes Bügelelement dar, wobei die Schenkelenden 62, 64 bogenförmig derart ausgebildet sind, daß sich Laschen ergeben, die ein einfaches Erfassen des Bügelelementes 58 gewährleisten. Mit anderen Worten müssen die bogenförmig ausgebildeten Enden 62 und 64 nur von zwei Fingern ergriffen werden, um die äußeren Schenkel des U-förmigen Bügelelementes aufeinanderzuzubewegen, so daß ein Entfernen aus den Längsschlitzen 60 möglich und damit ein Austausch bzw. ein Festlegen des Beutels 56 vorgenommen werden kann.

Die Funktion des Bügelelementes 58 sowie die Festlegung dieses im oberen Randbereich des Behälters 10 ist insbesondere der Fig. 4 und Fig. 5 zu entnehmen, so daß auf diese zeichnerischen Darstellungen nachdrücklich verwiesen wird.

## Patentansprüche

1. Hygienebehälter (10), beispielsweise zur Aufnahme von Binden, Tampons oder ähnlichem, der mit einem vorzugsweise eine verschwenkbare, eine Einfüllrutsche (18) aufweisende Klappe (22) umfassenden Deckel (12) verschließbar ist, wobei der Behälter (10) an zwei gegenüberliegenden Seiten (52) Einbuchtungen (34) aufweist, in denen Abschnitte (32) des Deckels (12) einbringbar sind,

dadurch gekennzeichnet,

daß die Einbuchtungen Führungen (34) sind, in denen die Abschnitte (32) verschiebbar sind, daß die Ränder oder zumindest Abschnitte der Ränder (28, 30, 42) des Deckels derart abgewinkelt sind, daß die Ränder bei auf dem Behälter aufgebrachtem Deckel fluchtend oder nahezu fluchtend zu den entsprechenden Seitenflächen (36, 52, 38) des Behälters ausgerichtet sind, und daß von der Deckelinnenfläche ein Arretierelement (44, 46) ausgeht, das in Form einer blattfederartigen Zunge mit gegebenenfalls abgewinkeltem freien Ende (46) im vorderen Bereich des Deckels (12) angeordnet ist, in den Behälter hineinragt und bei den Behälter verschließendem Deckel (12) im Bereich des Randes (48) der vorderen Seitenfläche (36) des Behälters derart zu liegen kommt, daß ein Herausziehen des Deckels unterbunden ist.

2. Hygienebehälter nach Anspruch 1,

dadurch gekennzeichnet,

daß jede Führung (34) eine im Abstand zum oberen Behälterrand (54) und parallel dazu verlaufende Ausnehmung vorzugsweise in Form einer Nut ist, die vorzugsweise im Abstand zur Rückwand (38) endet.

3. Hygienebehälter nach Anspruch 1 oder Anspruch 2,

dadurch gekennzeichnet,

daß die Ränder (28, 30, 42) des Deckels (12) in bezug auf die die Klappe (22) aufweisende Hauptfläche (20) abgewinkelt sind, wobei die Seitenränder (30) einander zugewandte, in die Führungen (34) einbringbare Abschnitte (32) aufweisen.

4. Hygienebehälter nach Anspruch 1,

dadurch gekennzeichnet,

daß der Behälter (10) an der Vorderfläche (36) einen stufenförmigen Absatz (50) aufweist, dessen Tiefen in etwa der Materialstärke des vorderen Randabschnitts (28) des Deckels (12) ist und dessen horizontaler Abschnitt im Bereich der unteren freien Kante des Randabschnitts (28) verläuft.

5. Hygienebehälter nach Anspruch 1 oder Anspruch 2, in dem ein Beutel entfernbar angeordnet ist,

dadurch gekennzeichnet,

daß der Beutel (56) von in zumindest zwei gegenüberliegenden Seiten des Behälters (10) lösbar angeordneten Halteelementen (58) festgelegt ist, die in zumindest zwei gegenüberliegenden, parallel oder nahezu parallel zur Behälteröffnung verlaufenden Längsschlitzen (60) eingebracht sind die ihrerseits durch die nach innen abgewinkelten parallel oder nahezu parallel zur Deckelfläche verlaufenden Behälterränder (54) und die parallel dazu in den zwei gegenüberliegenden Seiten verlaufenden, durch die Führungen für die Behälterdeckel-Randabschnitte vorgegebenen Einbuchtungen gebildet sind.

6. Hygienebehälter nach Anspruch 6,

dadurch gekennzeichnet,

daß das Halteelement (58) ein U-förmig ausgebildetes, federndes Bügelelement ist, dessen Seitenschenkel vorzugsweise in deren Endbereichen in das Behälterinnere erstreckende, als Griffe dienende Abschnitte (62, 64) aufweisen.

## Claims

1. A hygienical receptacle (10), for example to hold sanitary towels, tampons and the like, which is closable by a lid (12) preferably comprising a swing-type flap (22) having an intake chute (18), with the receptacle having on two opposite sides (52) recesses (34) into which sections (32) of the lid (12) are insertable,

characterized in that

the recesses are guideways (34) inside which the sections (32) are movable, that the edges or at least sections of the edges (28, 30, 42) of the lid are angled in such a way that the edges are aligned flush or almost flush with the respective lateral surfaces (36, 52, 38) of the receptacle when the lid is fitted to said receptacle, and that a locking element (44, 46) projects from the inner surface of the lid, said element being arranged in the form of leaf-spring-type tongue who se free end (46) is also angled if necessary in the front area of the lid (12), projecting into the receptacle, and, when the lid (12) closing the receptacle is near the edge (48) of the front lateral surface (36) of said receptacle, coming into contact in such a way that pulling out of the lid is prevented.

2. A hygienical receptacle according to Claim 1,

characterized in that

that each guideway (34) is a recess, preferably in the form of a groove, running at a distance from the upper edge (54) of the receptacle and parallel therwith and preferably terminating at a distance from the rear wall (38).

3. A hygienical receptacle according to Claim 1 or Claim 2,

characterized in that

the edges (28, 30, 42) of the lid (12) are angled in relation to the main surface (20) having the flap (22), with the lateral edges (30) having sections (32) facing each other and insertable into the guideways (34).

4. A hygienical receptacle according to Claim 1,

characterized in that

the receptacle (10) has a stepped section (50) whose depth is approximately that of the material thickness of the front edge section (28) of the lid (12) and whose horizontal section runs in the area of the lower free edge of the edge section (28).

5. A hygienical receptacle according to Claim 1 or Claim 2 in which a bag is removably arranged,

characterized in that

the bag (56) is held by detachably arranged holding elements (58) on at least two opposite sides of the receptacle (10), said elements being fitted in at least two opposite slots (60) running paralel or almost parallel to the receptacle opening, said slots in their turn being formed by the inwardly-angled receptacle edges (54) running parallel or almost parallel to the lid surface, and by the recesses running parallel thereto in the two opposite sides and created by the guideways for the receptacle lid edge sections.

6. A hygienical receptacle according to Claim 5, characterized in that the holding element (58) is a U-shaped, elastic bracket element whose legs preferably have sections (62, 64) at their ends protecting into the inside of the receptacle and serving as handles.

**Revendications**

1. Récipient hygiénique (10), destiné par exemple à recevoir des bandes, tampons ou similaires, qui peut être fermé hermétiquement à l'aide d'un couvercle enveloppant (12) lequel est doté de préférence d'un clapet basculant (22) présentant une glissière de remplissage (18), le récipient (10) présentant sur deux faces opposées (52), des évidements (34) dans lesquels des sections (32) du couvercle (12) peuvent être introduites,
caractérisé en ce que
ces évidements sont des glissières(34) dans lesquelles les sections (32) sont déplaçables, en ce que les bords ou au moins des sections des bords (28, 30, 42) du couvercle sont coudées, de telle manière que les bords, lorsque le couvercle est mis sur le récipient, soient alignés ou quasi-glignés sur les faces correspondantes (36, 52, 38) du récipient et qu'un élément de blocage (44, 46) parte de la face interne du couvercle, élément qui est disposé dans la partie avant du couvercle (12) sous forme d'une languette genre ressort à lames avec le cas échéant une extrémité libre coudée (46), s'engage dans le récipient et vient s'appliquer sur le couvercle (12) fermant le récipient, dans la zone du bord (48) de la face latérale avant (36), de façon à empêcher une extraction du couvercle.

2. Récipient hygiénique selon la revendication n° 1,
caractérisé en ce que
chaque glissière (34) est un évidement espacé du bord supérieur du récipient (54), qui suit un tracé parallèle à celui-ci et se présente de préférence sous la forme d'une rainure qui prend fin de préférence à une certaine distance de la paroi arrière (38).

3. Récipient hygiénique selon la revendication n° 1 ou 2,
caractérisé en ce que
les bords (28, 30, 42) du couvercle (12) sont coudés par rapport à la face principale (20) présentant le clapet (22), les bords latéraux (30) présentant des sections (32) orientées les unes vers les autres et pouvant s'engager dans les glissières (34).

4. Récipient hygiénique selon la revendication n° 1
caractérisé en ce que
le récipient (10) présente sur sa face avant (36) un retrait étagé (50) dont la profondeur correspond à peu près à l'épaisseur de matériau du bord avant (28) du couvercle (12) et dont la section horizontale se situe dans la zone de l'arête libre inférieure de la section du bord (28).

5. Récipient hygiénique selon la revendication n° 1 ou 2, dans lequel est disposé un sachet amovible,
caractérisé en ce que
le sachet (56) est fixé par des éléments de retenue détachables (58) disposés dans au moins deux faces opposées du récipient (10), éléments qui s'engagent dans au moins deux fentes longitudinales (60) suivant un tracé parallèle ou quasi-parallèle à l'orifice du récipient, fentes qui elles-mêmes sont formées par les bords de récipient (54) coudés vers l'intérieur ou suivant un tracé quasi-parallèle à la surface du couvercle et par les évidements, disposés parallèlement, situés dans les deux faces opposées et définis par les glissières destinées aux sections de bord du couvercle du récipient.

6. Récipient hygiénique selon la revendication n° 6
caractérisé en ce que
l'élément de retenue (58) est un élément élastique en forme d'étrier en U dont les joues latérales présentent, de préférence dans leurs zones extrêmes, des sections (62, 64) servant de poignées et s'étendant à l'intérieur du récipient.

FIG.1

FIG. 2

52

0 103 753

# FIG. 3

0 103 753

FIG.5

FIG.4